# EUROPEAN PATENT SPECIFICATION

(11) **EP 1 968 523 B1**
(45) Date of publication and mention of the grant of the patent: **15.02.2012**
(21) Application number: 05823017.8
(22) Date of filing: 23.12.2005
(51) Int. Cl.: A61J 3/10, A61K 9/20

(54) **PROCESS FOR PRODUCTION OF TABLETS**
VERFAHREN ZUR HERSTELLUNG VON TABLETTEN
PROCESSUS POUR PRODUIRE DES COMPRIMÉS

(43) Date of publication of application: 17.09.2008
(73) Proprietor: GEA Process Engineering A/S, 2860 Søborg (DK)
(72) Inventor: MADSEN, Michelle, Milling, DK-2660 Brøndby Strand (DK); SCHWARTZBACH, Henrik, DK-2760 MÅLØV (DK)
(74) Representative: Rasmussen, Torben Ravn
(86) International application number: PCT/DK2005/000829
(87) International publication number: WO 2007/071240

(56) References cited:
- EP-A- 1 295 595
- WO-A-01/22939
- GB-A- 1 287 431
- US-A- 4 904 477
- US-A- 5 670 167
- US-A- 6 098 895
- US-A1- 2003 042 639

## Description

### Introduction

The present invention relates to a process for producing tablets as well as a tablet comprising compressed particles.

### Background art

Tablets are one of the most frequently employed medicinal delivery forms offering a range of advantages for the producer as well as the patient. The producer of the tablet has the advantage of easy handling, packing and storing while the patient has the advantage of convenient administration.

In large scale production of tablets severe problems emerges with producing uniform tablets. For registration and safety purposes it is important that the variability of the various components in tablets is kept at a low level, so as to ensure that the patients receive the prescribed dosage and the physical properties of each tablet remains essentially constant.

Tablets are generally produced using one of the three processes: wet granulation, dry granulation, and direct compression. In wet granulation, components are typically mixed and granulated using a wet binder. The wet granulates are then sieved, dried and eventually grounded prior to compression into tablets. Wet granulation also includes melt granulation. In dry granulation, powder components are typically mixed prior to being compacted to yield hard slugs which are then ground and sieved before the addition of other ingredients. Finally, the particles are compressed into tablets.

Direct compression requires two steps only: dry mixing of all the ingredients and subsequent compression of this mixture into tablets. Direct compression is considered to be the simplest and the most economical process for producing tablets and is widely applied in the pharmaceutical industry. It has been estimated however, that less than 20 percent of pharmaceutical materials can be compressed directly into tablets (Shangraw, R. F., Compressed Tablets by Direct Compression Granualtion Pharmaceutical Dosage Forms: Tablets, Vol-1, Marcel Dekker, USA, 2nd ed., 195-246, 1989). The rest of the materials lack flow, cohesion, lubricating, or plastic deformation properties necessary for the production of tablets by direct compression.

For the fraction of pharmaceutical materials compressible by direct compression, the dry mixing of the components is a critical step that directly affects uniformity of the tablets produced. Generally, tumbling blenders are used for the mixing. A tumbling blender comprises a hollow contrainer attached to a rotating shaft and the tumbling blender is operated by partially filling the hollow container and rotating of the container. It is not possible to predict the mixing conditions for a uniform distribution of the components to be obtained, not even when a small scale or pilot scale mixing has been performed (Muzzio, F. J., Alexander, A. W.: Scale up of powder-blending operations, Blending and Mixing, Pharmaceutical Technology, March 2005). Therefore, if non-optimized mixing conditions are used the risk exist that the tablet quality is not sufficiently uniform.

It has been suggested to prepare an aqueous suspension of a dry blend comprising an active pharmaceutical ingredient and excipients and subsequent spray dry this suspension to obtain a powder having a mean particle diameter of 25µm (Viana, M., et al. Influence of Manufacture Process on Tabletting Ability of Powder: Comparison Between Blending, Grinding, and Spray Drying of Two Formulations Made of Theophylline and Lactose/Cellulose or Cellactose®, Drug Development and Industrial Pharmacy, 28(2), 119-127 (2002). The tablets were prepared by filling a die manually with an accurate mass of powder, and then subject the mass to an axial compression. This method results in tablets having a more uniform distribution of components. However, the prior art method is unsuitable for large-scale production of tablets due to impaired flowability of the obtained particles. The particles will have a tendency to stick together and will act as cohesive powders. This may be counteracted by using specialized fluidizing or transporting equipment and/or by adding other components to the particles to improve their flowability, which altogether makes the process more complex. However, in a high speed rotary tablet press filling of the dies is critical and very small particles are not suited well for this purpose.

US 4,904,477 discloses a spray dried ibuprofen composition suitable for direct compression into tablets comprising a spray dried dispersion in water of ibuprofen, pregalatinized starch, a disintegrant and a wetting agent for the ibuprofen.

EP 1 295 595 discloses a suspension that was spray dried in a spray dryer and a tablet was produced using a rotary tableting machine.

WO 01/22939 presents a method for preparing a spray-dried, compressible granular formulation for preparing pharmaceutical tablets.

US 4,016,254 presents microcapsules which have and average diameter of from 100 mu to 300 mu and which comprise 94% to 99.9% of a medicament coated by 0.1 % to 6% of a coating agent may be formed into a powder with 0% to 95% excipients or a tablet or capsule with a carrier.

US 4,600,579 discloses a method for preparing the essential components and spray drying the slurry.

US 4,631,284 discloses an acetaminophen composition containing a minor amount of a pheniramine maleate, a spray drying method for preparing the composition and orally admisterable analgesic antihistaminic tablets formed form the composition.

The present invention aims at designing a plant which produces flowable particles, enabling the use of an automated tablet press directly for production of tablets. As the particles comprise all essential components eventually appearing in the tablet, a dry mixing step may be omitted.

### Summary of the invention

The invention relates to a process for producing a tablet, comprising the steps as claimed in claim 1.

The process of the invention has one or more of the below-mentioned advantages over the direct compression method presently used in industry for producing tablets. First, the present method is easily scalable, i.e. moving from a research or pilot plant to a large-scale production facility does not necessitate comprehensive empirical tests to establish the mixing conditions. Second, the integration of the active pharmaceutical ingredient into the excipients at the liquid level will in most cases provide for less variability in the release profile. Third, the range of pharmaceutical compositions which can be prepared by compression of dried particles is increased. Fourth, a simplified method and a corresponding simplified plant for cost effectively production of tablets is devised.

The expression "all essential excipients" is intended to mean that further essential components are not added to the spray dried product before compression in a tablet press. Inessential components, which may be used to lubricate the tablet press, may be applied onto surfaces in contact with the particulate product. Traces of lubricant may then appear in the final product.

The expression "free-flowing particles" and equivalents thereto is intended to mean particles having sufficiently low interparticle cohesive forces for the particles to flow essentially independent of each other. A minor agglomeration of dry particles may be accepted as long as the process equipment and conduit lines are not clogged and the tablet press dies are filled satisfactory.

Prior to entering the spray dryer, the various components are mixed in a mixing device. The mixing device may be of the batch type or the continuous type. The batch type of mixing device generally comprises a container provided with agitation. The various components are added to the container sequentially or simultaneously under agitation or followed by agitation. Suitably, the liquid part(s) of the mixed liquid feed for the spray dryer is added to the container first and then the solid part(s) are added. The addition of the solid part(s) is generally performed in portions or slow enough for a proper dispersion of the components in the liquid. The agitation may be provided by any suitable means which can ensure a sufficient level of blending of the various components. As examples, the agitation may be provided by a propeller mounted on a rotating shaft or by shaking the container. The agitation is continued until the components are sufficiently blended, i.e. uniformly distributed in the mixture. The formed mixed liquid feed may be a solution, dispersion, suspension, or a mixture thereof.

For many practical approaches the mixed liquid feed is a suspension. The solvent may be selected to dissolute the active pharmaceutical ingredient, thereby allowing it to integrate into e.g. the structure of polymers and other excipients of the mixture. The amount of solvent is generally kept at a level at which the mixture is pumpable. The mixed liquid feed is conveyed to the atomizer of the spray dryer through a suitable conduit.

In a particular embodiment there may be more than one mixing device. In this case the mixed feeds may be combined immediately before entering the spray dryer or be fed to the spray dryer through separate nozzles. This is useful when components are incompatible as liquids or if the components require different solvents e.g. for their preparation.

Depending on the design of the apparatus of the invention, the atomizer may be selected from e.g. rotary atomizer wheel, two-fluid nozzle, three-fluid nozzle, four-fluid nozzle, ultrasonic atomizers, pressure nozzle, or a droplet generator. An example of a two-fluid nozzle is described in WO 2005/061119 (belonging to Niro A/S). An example of a rotary atomizer is described in EP 644802 (belonging to Niro A/S). An example of a special rotary atomizer, the Niro NARROWSPAN™ is described in EP 693972 (belonging to Niro A/S). An example of a droplet generator is described in WO 2005/061089 (belonging to Niro A/S)

The spray dryer comprises an inlet for drying air or gas. The drying gas is suitably entered into the drying chamber by a fan and a heater both capable of being adjusted to obtain a certain flow and temperature of the drying gas, i.e. supplying a predetermined drying capacity. The drying gas typically enters the drying chamber through an annular opening around the atomizer. It should be understood that spray drying includes spray cooling also. Further, the type of gas used is selected considering the solvent used. Also a supercritical fluid such as CO₂ may be used.

The drying chamber may have any suitable form, as long as the dried particles can be collected and transferred to the tablet press. Typically, the drying chamber comprises an upper part and a lower part, said upper part being essentially a cylinder closed in the top with a ceiling, and said lower part being a downward tapering frusto-conical wall. The downward tapering frusto-conical wall enters into an outlet for collecting the dried particles after the spray drying process. The particles withdrawn from the drying chamber may be classified in a cyclone.

The desired dried product may be transferred to an intermediate storage and then conveyed to the tablet press.

The drying chamber needs to be of a dimension which will allow droplets to dry to particles having a proportion between particle surface and weight which provides for the ability of the particles to flow freely and independently of each other. The ability of particles to flow independently is not solely determined by the surface/weight proportion, also the components of the composition and the processing conditions are of importance. Generally, the spray drying chamber needs to have a diameter of 0.5 m or more for allowing droplets of a size ensuring free flowing particles to be produced. It is desirable to keep the amount of solvent as low as possible, while maintaining a viscosity which makes the feed a-tomizable.

In a preferred embodiment, the drying chamber has a diameter of 1 m or more. In a still more preferred embodiment, the diameter is 2 m or more.

The plant of the invention may also comprise a device for secondary drying of the particles. The drying device may be selected from a variety of devices ready at hand for the skilled person. As examples, the drying device can be a moving endless belt for free or forced evaporation of the residual moisture, or a batch or continuous fluid bed. A fluid bed is generally preferred for better control of the final moisture content. A preferred continuous fluid bed is the Niro VIBRO-FLUIDIZER^{®}.

It may also be desired to cool the particles after the post-drying step. Suitably, then, the fluid bed is separated in a drying compartment and a cooling compartment for simultaneous drying and cooling of the particles. Also, pneumatic transport may be used for cooling of the particles.

In a certain aspect of the invention the fluid bed is an integral part of the spray dryer, e.g. as disclosed in EP 749560, EP 1227732, or in EP 961646 (all belonging to Niro A/S), the contents of which being incorporated herein in their entirety. The fluid bed is suitably provided in the lower conical part of the drying chamber and the dried particles are collected from the fluidized layer. An apparatus integrating spray drying and a fluid bed is typically chosen, when polymer excipients are included in the mixed fluid feed.

The exhausted drying gas may be filtered through an external filter or filter elements arranged internally in the drying chamber to separate the fine particles from the spent drying gas. The fine particles may be returned to the drying chamber when agglomerated particles are desired or the mixed liquid feed when individual particles are intended. Spray dryers having integrated filters in the drying chamber as disclosed in e.g EP 1227732, or in EP 961646 is particular appealing because the fine particles retained by the filters are subjected to a further agglomeration process when released from the filters.

If the fluid bed is integrated inside the spray dryer the still moist particles are collected in the bottom of the spray drying chamber. Primary hot drying gas is entered at the top and is used to dry the atomized particles. In addition a secondary gas flow is introduced at the bottom of the system to fluidise the agglomerated particles. The further agglomeration occurs in the fluidizing zone and relies on the moist surfaces of the particles to coalesce. This mechanism is dependant on residence time and particles concentration in this zone. Above the fluidised layer smaller particles are lifted into the chamber due to the fluidising air and are colliding with each other, forming a second agglomeration zone. According to the process of the invention, the various parameters able to be manipulated are adjusted so as to obtain the desired size and flowability of the individual or agglomerated particles.

The dried particles may be conveyed directly to the tablet press. However, it is generally preferable to transfer the dried particles to an intermediate storage for absorption of variations in the production. The intermediate storage device may comprise a container, a silo or the like. The particles may be conveyed between the various units used in the plant by known transportation means, including pneumatic means, screw conveyors, gravity, conveyor belts etc.

Tablet presses are available on the market from a variety of manufactures. A tablet press useful in the present invention includes a plurality of dies for accommodating particles and one or two punches movable in each die in an axial direction for compression of the tablet. In a certain aspect of the invention, the tablet press is a rotary tablet press, i.e. a tablet press comprising a rotatable die table comprising a series of dies arranged in the circumference. Each die is associated with at least one punch protruding into the die through an opening. The punch may be displayed axially by a cam when the table is rotated. A suitable tablet press are e.g. disclosed in US 6676863 (Courtoy).

A tablet press may be provided with a feeding device for automatic supply of dried particles to the dies. The feeding device communicates with an external supply channel delivering dried particles from the intermediate storage or directly from the spray dryer or fluid bed. The tablet press may be provided with means for applying a lubricant on the die and/or the tip(s) of the punch(es). Applicable means for applying lubricants comprises a screw conveyer, a venture or a pneumatic transport in order to blow lubricant onto the punches and/or the dies. According to an alternative method, the lubricant is sprayed onto the particles during the residence in a fluid bed or during pneumatic transport. However, in a certain aspect of the invention it is preferred to provide the surfaces of the compression chamber with a layer of lubricant because the used amount of lubricant can be lower for the same lubrication effect.

After the tablet has been compressed it is discharged and conveyed to a packing station. Optionally, the tablets are coated with a suitable coating before packing.

The invention also relates to a process. According to the process, the mixing is preferably conducted at a period of time and with a sufficient efficiency for an even distribution of the components to be obtained. The obtained mixed liquid feed may have any physical appearance, depending on the choice of active pharmaceutical ingredient, excipients, and solvent. Thus, the mixed liquid feed may be a solution, a dispersion, a suspension or an emulsion or a mixture thereof.

The components of the mixed feed may be selected from a variety of sources. Suitable examples of the solvent include water, ethanol, hexane, toluene, liquid CO₂, acetone, methylene chloride, and diethylether. Also, supercritical fluids may be used as solvents. Alternatively, the liquid feed is a melt with or without suspended solid material. When a volatile toxic solvent is used the plant is preferably provided with means for collecting the vapours and condensation thereof with a view of reusing the solvent.

The active pharmaceutical ingredient is not confined to a particular compound or group of compounds. Thus, the active pharmaceutical ingredient may be selected among e.g. antihypertensives, antianxiety agents, anticlotting agents, anticonvulsants, blood glucose-lowering agents, decongestants, antihistamines, antitussives, antineoplastics, beta blockers, anti-inflammatories, antipsychotic agents, cognitive enhancers, cholesterol-reducing agents, anti-atherosclerotic agents, antiobesity agents, autoimmune disorder agents, anti-impotence agents, antibacterial and antifungal agents, hypnotic agents, anti-Parkinsonism agents, anti-Alzheimer's disease agents, antibiotics, anti-depressants, antiviral agents, glycogen phosphorylase inhibitors, and cholesteryl ester transfer protein inhibitors.

Each named drug should be understood to include any pharmaceutically acceptable forms of the drug. By "pharmaceutically acceptable forms" is meant any pharmaceutically acceptable derivative or variation, including stereoisomers, stereoisomer mixtures, enantiomers, solvates, hydrates, isomorphs, polymorphs, pseudomorphs, neutral forms, salt forms and prodrugs.

A variety of excipients may be selected for producing the liquid feed resulting in the direct compressible tablets of the invention. Thus, the excipients may comprise one or more of the components selected from the group comprising binders, disintegrants, fillers, diluents, glidants, lubricants, flavours, sweeteners, and dyes. Specific examples of excipients include, but are not limited to, lactose, α-lactose monohydrate, anhydrous α-lactose, anhydrous β-lactose, microcrystalline cellulose (e.g. Avicel®), hydroxypropylcellulose, ethyl cellulose, sucrose, Di-Pac (97% sucrose and 3% modified dextrin), Nu-tab (sucrose, invert sugar, corn starch and magnesium stearate), Emdex (hydrolysed starch), Starch 1500 (partially hydrolized cornstarch), mannitol, starch, maltose, dicalcium phosphate anhydrate (e.g. Fujicalin), dicalcium phosphate (e.g.Emcompress), sorbitol, lactitol (e.g. Finlac DC), xylitol, maltodextrin, powdered cellulose, tricalcium phosphate, calcium sulphate, calcium lactate pentahydrate, calcium lactate trihydrate, aluminium hydroxide, polyvinyl pyrrolidone, crospovidone, inulin, Ludipress (93.4% α-lactose monohydrate, 3.2% polyvinyl pyrrolidone (Kollidon 30) and 3.4% crospovidone (Kollidon CL)), Cellactose (75% α-lactose monohydrate and 25% cellulose), Pharmatose DCL 40 (95% β-lactose and 5% anhydrous lactitol), Prosolv (silicified microcrystalline cellulose), and StarLac (lactose monohydrate and maize starch). A variety of further polymers is contemplated, such as HPMC, HPMCAS, HPMCP, CAP, CAT, carboxyethyl cellulose, carboxymethyl cellulose, ethylcarboxymethyl cellulose, PLG, PLA, PLGA, etc.

A lubricant may also be included in the liquid feedstock or alternatively sprayed onto the particles upstream of the tablet press or used to cover the surfaces of the compression chamber. Suitable examples of a lubricant include magnesium stearate, stearic acid, calcium stearate, and talc.

The process conditions of the spray dryer are adjusted so as to obtain particles with a mean diameter (D₅₀) of 40µm or above. A characteristic feature of desired particles is that the interparticle cohesive forces are small enough for the particles to flow essentially independent of each other. At a mean diameter of 40-70µm the interparticle cohesive force generally is so reduced relative to the weight that an independent flow of each particle is obtained. A better flowability may be obtained for particles having a mean diameter of 100µm or above. For some applications the mean particle size is in the range of 200-600µm.

The tablet press used in accordance with the present process comprises a die into which the essentially free flowing particles are filled. The die may include means for varying the volume of the die for compensate for any variation in the density of the particles mass. After the filling, the particles are compressed using a suitable force. Typically, a compression pressure of 40 to 400 MPa is used. In a preferred embodiment, the tablet press is of the rotary tablet press type and the particles are automatic feed into the die thereof. To avoid clogging in the feeding system flowability of the particles are of particular importance. While a minor cohesion of the individual particles may be accepted, it is essential for the process of the invention to work properly that the particles are of size and possesses surface characteristics which will not severely clog the system.

After the tablets have been pressed they may or may not be coated. The aim of coating tablets may be to make them smoother, provide them with a certain colour, make them less unpleasant to swallow, or to protect the content of the tablet during storage. Certain tablets are provided with a so-called enteric coating, which will prevent the tablet from being disintegrated in the acidic environment of the stomach so as to deliver the active principle in the intestine. Enteric coatings are particular suitable for tablets containing active pharmaceutical ingredients easy degradable in acid.

The invention also relates to a tablet comprising compressed particles, wherein each particle contains the active pharmaceutical ingredient(s) and all essential excipients of the tablet, said particles prior to compression having a mean diameter of 40µm or more.

In a certain aspect of the invention, the particles are of a mean diameter of 70µm, suitably 100µm, or above. The tablet is produced by compression of a particle mass with a force sufficient for the particles to form a self-supporting body. Suitably, the compression is performed using a pressure in the range of 40 to 400 MPa. A higher compression force may be used for special applications requiring a high coherence of the particles.

According to the invention, each of the particles contains all the essential components of the tablet. This is opposed to the traditional direct compression of tablets in which the individual components appears as separate particles. The uniform distribution of the individual components in each particle ensures that the tablets show less variability and thus that the intended dosage is accurate, provided the mixed liquid feed for spray drying is thoroughly blended, i.e. mixed to an extend in which the individual component are essentially uniformly distributed in the liquid. The invention also offers the possibility of adjusting the release profile using various excipients for retarding or promoting the release. Thus, the chemical nature of the excipients may be used to alter the release profile of the active pharmaceutical ingredient.

### Example (reference example)

### Preparation of a tablet

A liquid feed was prepared in a container provided with agitation. The container was loaded with 64 l water and the following components were added slowly while constantly stirring:

| | |
|---|---|
| Paracetamol | 24 kg |
| Lactose Monohydrate | 7 kg |
| Micro Crystalline Cellulose | 3 kg |
| Maize Starch | 1 kg |
| Mg-Stearate | 1 kg |

The resulting dispersion was pumped to a spray dryer (Niro SD-12.5 having diameter of 2,5 m) and atomized using a rotary nozzle wheel with a diameter of 100 mm and a velocity of 20.000 rpm. The feed rate was 42 kg/h and the spray dryer inlet temperature of the drying gas was adjusted to 190°C. The spray dryer outlet temperature was adjusted to 100°C and the amount of drying gas (air) was 1250 kg/h.

The dried particles were withdrawn from the drying chamber and collected by a cyclone. The particles collected by the cyclone had a mean diameter (D₅₀) of 70 µm. The product rate was 16 kg/h.

The residual moisture of the particles was < 6 wt % and the particles displayed a spherical morphology when observed in microscope. The flowability was satisfactory having an angle of repose of 30°. The density of the powder was 0.40 g/cm³.

The particles were charged to a Courtoy rotary tablet press MODUL™ and pressed into tablets having a compact strength of 180-200N.

## Claims

1. A process for producing tablets, comprising the steps of
mixing a solvent, active pharmaceutical ingredient(s), and all essential excipients appearing in the tablet for producing a mixed liquid feed,
spray drying the mixed liquid feed into dried individual or agglomerated particles having a mean diameter of 40µm or more,
automatically feeding the dried particles into a rotary tablet press comprising a plurality of dies for accommodating particles and one or two punches axially movable in each die, and
compressing the particles into a tablet by moving the punch(es) in the die,
wherein the die and/or the tip(s) of the punch(es) have been applied a lubricant or the particles upstream of the rotary tablet press have been sprayed with a lubricant.

2. The process according to claim 1, wherein the particles have a mean diameter of 70µm or more.

3. The process according to claim 1 or 2, wherein the particles have a mean diameter of 100µm or more.

4. The process according to any of the claims 1 to 3, wherein dried particles are subjected to fluid bed processing to obtain a secondary drying.

5. The process according to any of the claims 1 to 4, wherein the excipients comprise one or more of the components selected from the group comprising binders, disintegrants, fillers, diluents, flavours, sweeteners, and dyes.

6. The process according to any of the claims 1 to 5, wherein the compression of the tablet is performed by filling a die of a rotary press with a desired amount of dried particles and compressing the particles mass in the die using a pressure in the range of 40 to 400 MPa.

7. The process according to claims 1 to 6 further comprising covering the tablet with a coating.

8. A tablet comprising compressed particles, wherein the particles have been produced by spray drying of a mixed liquid feed comprising a solvent, active pharmaceutical ingredient(s) and all essential excipients of the tablet, and wherein the die and/or the tip(s) of the punch(es) have been applied a lubricant or a lubricant has been sprayed onto the particles upstream of a tablet press, said particles prior to compression having a mean diameter of 40µm or more.

9. The tablet according to claim 8, wherein the mean diameter of the dried particles is 70µm or more.

10. A tablet according to any of the claims 8 or 9, wherein the mean diameter of the dried particles is 100µm or more.

11. The tablet according to any of the claims 8 to 10, wherein the compression is performed using a pressure in the range of 40 to 400 MPa.

12. A tablet according to any of the claims 8 to 11, further comprising a coating.

## Patentansprüche

1. Verfahren zur Herstellung von Tabletten, das die folgenden Schritte umfasst:
Mischen eines Lösungsmittels, eines oder mehrerer pharmazeutischer Wirkstoffe und aller wesentlichen Hilfsstoffe, die in der Tablette vorkommen, zur Herstellung eines gemischten flüssigen Zuführungsprodukts,
Sprühtrocknen des gemischten flüssigen Zuführungsprodukts zu getrockneten einzelnen oder agglomerierten Teilchen, die einen mittleren Durchmesser von 40 µm oder mehr haben,
automatisches Zuführen der getrockneten Teilchen in eine Rotations-Tablettenpresse, die eine Mehrzahl von Pressformen zum Aufnehmen der Teilchen und einen oder zwei Stanzer, die in jeder Pressform axial beweglich sind, umfasst, und
Komprimieren der Teilchen zu Tabletten durch Bewegen des oder der Stanzer in der Pressform,
worin die Pressform und/oder die Spitze(n) des oder der Stanzer mit einem Schmiermittel versehen wurden oder die Teilchen, der Rotationstablettenpresse vorgeschaltet, mit einem Schmiermittel besprüht wurden.

2. Verfahren gemäß Anspruch 1, worin die Teilchen einen mittleren Durchmesser von 70 µm oder mehr aufweisen.

3. Verfahren gemäß Anspruch 1 oder 2, worin die Teilchen einen mittleren Durchmesser von 100 µm aufweisen.

4. Verfahren gemäß irgendeinem der Ansprüche 1 bis 3, worin die getrockneten Teilchen einem Fließbettverfahren unterzogen werden, um ein sekundäres Trocknen zu erreichen.

5. Verfahren gemäß irgendeinem der Ansprüche 1 bis 4, worin die Hilfsstoffe eine oder mehrere Komponenten, die aus Bindemittel, Disintegrationsmittel, Füllstoffen, Verdünnungsmitteln, Geschmacksstoffen, Süßungsmitteln und Farbstoffen ausgewählt sind, umfassen.

6. Verfahren gemäß irgendeinem der Ansprüche 1 bis 5, worin die Komprimierung der Tablette durchgeführt wird, indem eine Pressform der Rotationspresse mit einer gewünschten Menge der getrockneten Teilchen gefüllt wird, und die Teilchenmasse in der Pressform unter Verwendung eines Drucks im Bereich von 40 bis 400 MPa komprimiert wird.

7. Verfahren gemäß irgendeinem der Ansprüche 1 bis 6, das weiterhin das Bedecken der Tablette mit einer Beschichtung umfasst.

8. Tablette, die komprimierte Teilchen umfasst, worin die Teilchen durch Sprühtrocknen eines gemischten flüssigen Zufuhrerzeugnisses hergestellt wurden, das ein Lösungsmittel, einen oder mehrere pharmazeutische Wirkstoffe und alle wesentlichen Hilfsstoffe der Tablette umfasst, und worin die Pressform und/oder die Spitze(n) des oder der Stanzer mit einem Schmiermittel versehen wurden oder ein Schmiermittel der Tablettenpresse vorgelagert auf die Partikel gesprüht wurde, wobei die Partikel vor dem Komprimieren einen mittleren Durchmesser von 40 µm oder mehr aufweisen.

9. Tablette gemäß Anspruch 8, worin der mittlere Durchmesser der getrockneten Partikel 100 µm oder mehr ist.

10. Tablette gemäß irgendeinem der Ansprüche 8 oder 9, worin der mittlere Durchmesser der getrockneten Partikel 100 µm oder mehr ist.

11. Tablette gemäß irgendeinem der Ansprüche 8 bis 10, worin die Kompression unter Verwendung eines Drucks im Bereich von 40 bis 400 MPa durchgeführt wird.

12. Tablette gemäß irgendeinem der Ansprüche 8 bis 11, die weiterhin eine Beschichtung umfasst.

## Revendications

1. Procédé pour produire des comprimés, comprenant les étapes consistant à :
mélanger un solvant, un ou plusieurs ingrédients pharmaceutiques actifs, et tous les excipients essentiels apparaissant dans le comprimé, pour produire une charge liquide mixte,
sécher par pulvérisation la charge liquide mixte en particules individuelles ou agglomérées séchées ayant un diamètre moyen de 40 µm ou plus,
introduire automatiquement les particules séchées dans une presse à comprimés rotative comprenant une pluralité de matrices pour loger les particules et un ou deux poinçons axialement mobiles dans chaque matrice, et
compresser les particules en un comprimé par déplacement du ou des poinçons dans la matrice,
dans lequel un lubrifiant a été appliqué sur la matrice et/ou la ou les pointes du ou des poinçons ou bien les particules en amont de la presse à comprimés rotative ont été pulvérisées avec un lubrifiant.

2. Procédé selon la revendication 1, dans lequel les particules ont un diamètre moyen de 70 µm ou plus.

3. Procédé selon la revendication 1 ou 2, dans lequel les particules ont un diamètre moyen de 100 µm ou plus.

4. Procédé selon l'une quelconque des revendications 1 à 3, dans lequel les particules séchées sont soumises à un traitement en lit fluide pour que soit obtenu un séchage secondaire.

5. Procédé selon l'une quelconque des revendications 1 à 4, dans lequel les excipients comprennent un ou plusieurs composants choisis dans l'ensemble constitué par les liants, les délitants, les charges, les diluants, les arômes, les édulcorants, et les colorants.

6. Procédé selon l'une quelconque des revendications 1 à 5, dans lequel la compression du comprimé est effectuée par remplissage d'une matrice d'une presse rotative avec une quantité souhaitée de particules séchées, et compression de la masse de particules dans la matrice au moyen d'une pression située dans la plage allant de 40 à 400 MPa.

7. Procédé selon l'une quelconque des revendications 1 à 6, comprenant en outre l'enrobage du comprimé avec un enrobage.

8. Comprimé comprenant des particules compressées, dans lequel les particules ont été produites par séchage par pulvérisation d'une charge liquide mixte comprenant un solvant, un ou plusieurs ingrédients pharmaceutiques actifs et tous les excipients essentiels du comprimé, et dans lequel un lubrifiant a été appliqué sur la matrice et/ou la ou les pointes du ou des poinçons ou bien un lubrifiant a été pulvérisé sur les particules en amont d'une presse à comprimés, lesdites particules, avant compression, ayant un diamètre moyen de 40 µm ou plus.

9. Comprimé selon la revendication 8, dans lequel le diamètre moyen des particules séchées est de 70 µm ou plus.

10. Comprimé selon l'une quelconque des revendications 8 et 9, dans lequel le diamètre moyen des particules séchées est de 100 µm ou plus.

11. Comprimé selon l'une quelconque des revendications 8 à 10, dans lequel la compression est effectuée au moyen d'une pression située dans la plage allant de 40 à 400 MPa.

12. Comprimé selon l'une quelconque des revendications 8 à 11, comprenant en outre un enrobage.
